# EUROPÄISCHE PATENTSCHRIFT

(11) **EP 0 334 282 B1**
(45) Veröffentlichungstag und Bekanntmachung des Hinweises auf die Patenterteilung: **13.07.1994**
(21) Anmeldenummer: 89105012.2
(22) Anmeldetag: 21.03.1989
(51) Int. Cl.: C12N 15/00, C12N 1/20

(54) **Verwendung von pSG5 als temperatursensitives Plasmid**
Use of pSG5 as a temperature-sensitive plasmid
Utilisation du pSG5 comme plasmide thermosensible

(30) Priorität: 23.03.1988 DE 3809692
(43) Veröffentlichungstag der Anmeldung: 27.09.1989
(73) Patentinhaber: HOECHST AKTIENGESELLSCHAFT, 65926 Frankfurt am Main (DE)
(72) Erfinder: Wohlleben, Wolfgang, Dr., D-4800 Bielefeld (DE); Muth, Günter, Dr., D-4800 Bielefeld (DE); Pühler, Alfred, Prof. Dr., D-4800 Bielefeld (DE); Nussbaumer, Bernhard, D-4900 Herford (DE)

(56) Entgegenhaltungen:
- EP-A- 0 158 201
- EP-A- 0 158 872
- EP-A- 0 243 856
- EP-A- 0 257 416
- THE JOURNAL OF GENERAL MICROBIOLOGY, Band 131, Teil 6, Juni 1985, Seiten 1299-1303, SGM, GB; A.W. BIRCH et al.: "Temperature-sensitive mutants of the Streptomyces plasmid pIJ702"
- MOL. GEN. GENET., Band 211, 1988, Seiten 424-429, Springer-Verlag; G. MUTH et al.: "The minimal replicon of the Streptomyces ghanaesis plasmid pSG5identified by subcloning and Tn5 mutagenesis"

## Beschreibung

Aus der europäischen Patentschrift (EP-B) Nr. 0 158 872 ist das Streptomyceten-Plasmid pSG5 bekannt, das aus einer Kultur von Streptomyces ghanaensis DSM 2932 isoliert werden kann. Dieses Plasmid eignet sich zur Herstellung von Hybridvektoren, beispielsweise für sogenannte Pendelvektoren, die aufgrund eines eingebauten E. coli-Replikons in E. coli-Stämmen vermehrt werden können. Solche Vektoren sind beispielsweise aus der europäischen Patentanmeldung mit der Veröffentlichungs-Nr. (EP-A) 158 201 bekannt.

Es wurde nun gefunden, daß pSG5 temperatursensitiv ist.

Die Eigenschaft der Temperatursensitivität für pSG5 ist überraschend, da bisher noch keine natürlichen Streptomyceten-Plasmide bekannt wurden, die diese Eigenschaft haben. Da pSG5 und die mit seinem Replikon hergestellten Hybridplasmide einen breiten Wirtsbereich aufweisen, eröffnet die erfindungsgemäße neue Verwendung dieses Plasmids dem Fachmann eine Fülle von Möglichkeiten:
Wenn man ein Plasmid mit dem Replikon von pSG5 konstruiert, das einen Marker enthält, transformiert damit einen kompatiblen Wirtsstamm und erhöht dann die Temperatur über den Schwellenwert, so werden nach Selektion auf den Marker nur solche Transformanten erhalten, die das Plasmid ganz oder teilweise in das Genom integriert haben. Da solche Integrationen im wesentlichen nur in homologe Bereiche des Genoms erfolgen, eignet sich dieses Verfahren zum Auffinden solcher homologer Bereiche im Genom des Wirtsstammes.

Aus der EP-A 0 243 856 ist ein Verfahren zur Herstellung von Mutanten bekannt, das dadurch gekennzeichnet ist, daß man aus dem Ausgangsstamm die gesamte DNA isoliert, sie in kurze Fragmente überführt, diese in ein Plasmid integriert, das einen Marker enthält, temperatursensitiv ist und in dem Ausgangsstamm repliziert, die erhaltene Hybrid-Population in den Ausgangsstamm transformiert, durch Selektion auf den Marker die Transformanten selektiert, durch Erhöhung der Temperatur über den Schwellenwert des temperatursensitiven Plasmids die Hybridplasmide eliminiert und durch erneute Selektion auf den Marker die Mutanten selektiert.

Der Begriff "kurze" Fragmente bedeutet DNA-Abschnitte, die mit häufig schneidenden Restriktionsenzymen wie Sau3A oder TaqI, aber auch mit mechanischen Methoden (Ultraschall, Scheren) erhalten werden, und die weder die Promotorregion noch die Signale für den Translationsstop enthalten.

Da nach Elimination der Plasmide bei einer Selektion auf den Marker nur Zellen überleben, die Plasmid-DNA in ihr Chromosom aufgenommen haben (was bevorzugt über die in dem Plasmid integrierte homologe DNA erfolgt), werden unmittelbar die Mutanten erhalten.

Das Plasmid pSG5 ist in der EP-A 0 243 856 als geeignetes Ausgangsplasmid aufgeführt. Erfindungsgemäß kann jedoch nunmehr beim Einsatz dieses Plasmids die Mutation zu temperatursensitiven Replikationsmutanten entfallen. Damit erspart man sich nicht nur die Mutation und die besonders aufwendige Selektion, sondern man vermeidet auch die Gefahr, unerwünschte Mehrfachmutationen zu erzeugen. Das Plasmid pSG5 ist also für dieses Verfahren besonders gut geeignet.

Die Temperatursensitivität von pSG5 äußert sich so, daß dieses Plasmid bei Temperaturen ab 36°C instabil wird und nicht mehr repliziert. Die Obergrenze des nutzbaren Temperaturbereichs ist von der Wirtszelle abhängig: In S. venezuelae liegt sie beispielsweise bei 38°C, in S. lividans bei 39°C und bei S. ghanaensis bei 45°C. Werden Kulturen, die pSG5 oder einen Abkömmling dieses Plasmids enthalten, bei einer Temperatur von 36°C oder darüber inkubiert, so "verdünnt sich das Plasmid heraus" und ist nach wenigen Generationen nicht mehr nachweisbar.

Die erfindungsgemäße Verwendung des pSG5-Replikons kann also dafür genutzt werden, homologe Gene zu einem vorhandenen Gen aufzufinden. Man hat so eine brauchbare Alternative zur Anlage einer Genbank und "Screening" mit markierten DNA-Sonden. Diese Alternative ist insbesondere dann wertvoll, wenn durch Hybridisierung kein aussagekräftiges Ergebnis erhalten wurde. Ein weiterer Vorteil dieser Methode ist, daß das Arbeiten mit radioaktiven Substanzen vermieden werden kann.

Entsprechend können auch Insertionselemente (IS-Elemente) und Transposons aufgefunden werden, die in das Genom des untersuchten Wirtsstammes aufgenommen wurden: Verwendet man nämlich das nur mit dem Marker versehene Plasmid ohne weitere inserierte DNA, so stehen homologe Bereiche nur dann zur Verfügung, wenn vor der Temperaturerhöhung ein IS-Element oder Transposon vom Chromosom auf das Plasmid übertragen wurde. Damit ermöglicht die Selektion auf den Marker das Auffinden solcher DNA-Elemente.

Die genannten Untersuchungen können entsprechend dem Verfahren zur Isolierung von Mutanten nach der EP-A 0 243 856 erfolgen.

Die Erfindung bringt somit eine Reihe von Vorteilen mit sich:
1. Auf der Grundlage des pSG5-Plasmids existiert bereits eine wegen des breiten Wirtsbereichs vielseitig anwendbare Vektorfamilie mit verschiedenen Selektionsmarkern wie Resistenz gegen Neomycin, Thiostrepton, Kanamycin (EP-A 0 158 201) und Gentamicin (EP-A 0 248 207) sowie Farbmarkern wie Melanin und andere Farbstoffe bzw. Pigmente (EP-A 0 257 416 und 0 257 417).
2. Benutzt man aus der genannten Vektor-Familie ein Plasmid mit einem Marker, der in E. coli selektionierbar ist, so kann das aus der EP-A 0 243 856 bekannte Verfahren auf den Einsatz von Cosmid-Banken und damit die Isolierung großer DNA-Abschnitte von etwa 40 kb ausgedehnt werden: Wird der in E. coli selektionierbare Marker in das Chromosom des Wirts integriert, so kann das Genom der so entstandenen Mutante in eine Cosmid-Genbank überführt werden. Die Selektion auf den Marker (zweckmäßig gleichzeitig mit dem Cosmid-eigenen Marker) führt dann unmittelbar zu dem interessierenden Cosmid-Klon. Man erspart so das bisher erforderliche, äußerst aufwendige "Screening" durch Hybridisierung. Im Gegensatz zu dem aus der EP-A 0 243 856 bekannten Verfahren kann man so in einem Schritt große Genregionen in der Nachbarschaft zu dem mutierten Gen erfassen. Man kann auf diese Weise Gen-"Cluster" isolieren, also beispielsweise die Gene für einen ganzen Biosyntheseweg. Man ist auch nicht mehr darauf angewiesen, daß in der Nähe des mutierten Gens geeignete Schnittstellen für Restriktionsenzyme vorhanden sind.

In den folgenden Beispielen wird die Erfindung näher erläutert. Prozentangaben beziehen sich hierbei auf das Gewicht, wenn keine anderen Angaben gemacht sind.

### Beispiel 1: Gesamt-DNA-Isolierung

0,1 g Mycel einer 3 Tage alten, homogenisierten Streptomycetenkultur des Stammes S. ghanaensis (ATCC 14672; US-PS 3 674 866), der kein Melanin produziert (mel⁻) werden im 1,5 ml Eppendorf-Reaktionsgefäß in einer Eppendorf-Zentrifuge 1 min pelletiert und dann einmal mit 0,5 ml TE (10 mM Tris-HCl, 1 mM EDTA (pH 8) mit 10 % Saccharose) gewaschen. Das Pellet wird dann in 0,5 ml Lysozymlösung (0,3 M Saccharose, 25 mM Tris-HCl (pH 8), 25 mM EDTA, 10 mg/ml Lysozym) resuspendiert und 60 min bei 37°C inkubiert. Nach Zugabe von 0,2 ml 5 %iger SDS-Lösung und Durchmischen der Lösung wird diese 10 min bei 65°C inkubiert und danach wieder auf Raumtemperatur abgekühlt. Anschließend gibt man 100 »l Phenol/Chloroform (5 g Phenol, 5 ml Chloroform, 5 mg 8-Hydroxychinolin, 1 ml 0,1 M Tris (pH 8)) zu und mischt vorsichtig auf einem Schüttler (^{(R)}Vortex), bis die Suspension homogen ist. Danach wird das Gemisch 5 min in einer Eppendorf-Zentrifuge zentrifugiert und die obere, wäßrige Phase in ein neues Reaktionsgefäß überführt. Der DNA-haltigen Lösung wird 70 »l 3 M ungepuffertes Na-acetat und 700 »l Isopropanol zugesetzt. Nach Mischen und 15-minütiger Inkubation bei Raumtemperatur wird die DNA durch Zentrifugation (5 min in Eppendorf-Zentrifuge) pelletiert und der Überstand quantitativ entfernt. Die DNA resuspendiert man in 300 »l TE und inkubiert sie dann 45 min bei 37°C mit 10 »l RNase-Lösung (50 »g RNase/ml H₂O). Die RNase wird durch 100 »l Phenol/Chloroform inaktiviert und die denaturierten Proteine pelletiert (5 min in Eppendorf-Zentrifuge). Die DNA-haltige Lösung wird erneut mit Isopropanol behandelt (Zugabe von 30 »l 3 M Na-acetat und 400 »l Isopropanol, 15 min Inkubation bei Raumtemperatur). Das DNA-Pellet, das man nach der Zentrifugation erhält, wird zweimal mit 70 %igem Ethanol gewaschen und erneut pelletiert. Nach Trocknen nimmt man die DNA in 300 »l TE auf und verwendet sie für die weiteren Schritte.

### Beispiel 2: Spaltung der Gesamt-DNA mit Sau3A

1 »g DNA wird im Spaltungspuffer (50 mM Tris-HCl (pH 8), 10 mM MgCl₂, 50 mM NaCl) in Gegenwart von 1 unit Sau3A (Hersteller: BRL-Gibco, Karlsruhe) 1 h bei 37°C inkubiert. Die Reaktion wird durch Phenolisierung gestoppt und die DNA durch Ethanolfällung gereinigt.

### Beispiel 3: Klonierung von Gesamt-DNA-Fragmenten in das Plasmid pGM4

pGM4 (EP-A 0 257 416 und 0 257 417) wird analog Beispiel 2 mit BamHI vollständig linearisiert. Die beiden DNA-Proben werden im Spaltungspuffer gemischt, auf 70°C erhitzt und durch Zugabe von Mercaptoethanol (Endkonz. 10 mM) und ATP (0,1 mM) auf die Ligase-Reaktionsbedingungen eingestellt. In Gegenwart von 1 unit T4-DNA-Ligase (Boehringer Mannheim) wird das Gemisch 12 h bei 14°C inkubiert. Anschließend wird das Gemisch in Protoplasten des Ausgangsstammes transformiert und auf Regenerationsplatten ausplattiert. Diese werden nach etwa 20 h mit Weichagar überschichtet, der soviel Thiostrepton enthält, daß die Endkonzentration in der Platte 50 »g/ml beträgt.

### Beispiel 4: Erzeugung und Selektion von Mutanten

Die Transformanten werden in einer Schüttelkultur in S-Medium (Hopwood et al., "Genetic Manipulation of Streptomyces, a Laboratory Manual", The John Innes Foundation, Norwich 1985) ca. 36 h bei 28°C inkubiert. Anschließend wird die Temperatur auf 39°C erhöht und nochmals ca. 36 h bei dieser Temperatur inkubiert. Die Kultur wird steril geerntet, in TE + 10 % Saccharose gewaschen und in Vollmedium mit Thiostrepton (20 mg/l) weitere 48 h bei 39°C inkubiert. Anschließend werden die so erzeugten Mutanten ausplattiert und charakterisiert (Inkubation stets bei 39°C).

### Beispiel 5: Isolierung der mutierten DNA

Aus den Mutanten wird gemäß Beispiel 1 die DNA isoliert, mit einem Restriktionsenzym, das keine Schnittstellen im integrierten Plasmid besitzt, geschnitten und mit T4-DNA-Ligase religiert. Hierdurch wird das integrierte Plasmid, das jetzt das mutierte Gen enthält, als einzige replikationsfähige cyclische DNA gebildet. Nach Retransformation in einen geeigneten Stamm wie S. lividans selektiert man auf Thiostrepton-Resistenz und isoliert aus den Transformanten das Plasmid, das das mutierte Gen trägt.

## Patentansprüche (Patentansprüche für folgende(n) Vertragsstaat(en): AT, BE, CH, LI, DE, FR, GB, GR, IT, LU, NL, SE)

1. Verwendung des pSG5-Replikons zur Herstellung temperatursensitiver Hybridplasmide.

2. Verfahren zur Auffindung von IS-Elementen und Transposons, dadurch gekennzeichnet, daß man ein Plasmid, das das pSG5-Replikon enthält, mit einem Marker versieht, mit dem so erhaltenen Hybridplasmid Streptomyceten transformiert, die Transformanten bei einer Temperatur oberhalb von 36°C inkubiert, auf den Marker selektiert und den DNA-Bereich analysiert, über den die Rekombination erfolgte.

3. Verfahren zur Herstellung von Streptomyceten-Mutanten, zur Isolierung der mutierten Gene und zur Isolierung des entsprechenden Wildtyp-Gens, dadurch gekennzeichnet, daß man aus dem Streptomyceten-Ausgangsstamm die gesamte DNA isoliert, sie in kurze Fragmente überführt, diese in ein Hybridplasmid mit dem pSG5-Replikon integriert, das einen Marker enthält, die erhaltenen Hybridplasmid-Population in den Ausgangsstamm transformiert, durch Selektion auf den Marker die Transformanten selektiert, durch Erhöhung der Temperatur über den Schwellenwert die Hybridplasmide eliminiert und durch erneute Selektion auf den Marker die Mutanten selektiert.

4. Verfahren nach Anspruch 3, dadurch gekennzeichnet, daß aus den selektierten Mutanten die mutierten Gene isoliert werden.

5. Verfahren nach Anspruch 4, dadurch gekennzeichnet, daß mit Hilfe der mutierten Gene die entsprechenden Wildtyp-Gene isoliert werden.

6. Verfahren nach Anspruch 4, dadurch gekennzeichnet, daß das Hybridplasmid mit dem pSG5-Replikon einen in E. coli selektionierbaren Marker enthält und daß bei der Isolation der mutierten Gene eine Cosmid-Bank eingesetzt wird.

## Patentansprüche (Patentansprüche für folgende(n) Vertragsstaat(en): ES)

1. Verfahren zur Herstellung von temperatursensitiven Hybridplasmiden, dadurch gekennzeichnet, daß man DNA, die das Replikon von pSG5 enthält, mit fremder DNA ligiert.

2. Verfahren nach Anspruch 1, dadurch gekennzeichnet, daß die fremde DNA einen Marker enthält.

3. Verfahren nach Anspruch 1 oder 2, dadurch gekennzeichnet, daß die fremde DNA ein linearisiertes Plasmid ist.

4. Verfahren zur Herstellung von Streptomyceten-Mutanten, zur Isolierung der mutierten Gene und zur Isolierung des entsprechenden Wildtyp-Gens, dadurch gekennzeichnet, daß man aus dem Streptomyceten-Ausgangsstamm die gesamte DNA isoliert, sie in kurze Fragmente überführt, diese in ein Hybridplasmid mit dem pSG5-Replikon integriert, das einen Marker enthält, die erhaltene Hybridplasmid-Population in den Ausgangsstamm transformiert, durch Selektion auf den Marker die Transformanten selektiert, durch Erhöhung der Temperatur über den Schwellenwert die Hybridplasmide eliminiert und durch erneute Selektion auf den Marker die Mutanten selektiert.

5. Verfahren nach Anspruch 4, dadurch gekennzeichnet, daß aus den selektierten Mutanten die mutierten Gene isoliert werden.

6. Verfahren nach Anspruch 5, dadurch gekennzeichnet, daß mit Hilfe der mutierten Gene die entsprechenden Wildtyp-Gene isoliert werden.

7. Verfahren nach Anspruch 5, dadurch gekennzeichnet, daß das Hybridplasmid mit dem pSG5-Replikon einen in E. coli selektionierbaren Marker enthält und daß bei der Isolation der mutierten Gene eine Cosmid-Bank eingesetzt wird.

8. Verfahren zur Auffindung von IS-Elementen und Transposons, dadurch gekennzeichnet, daß man pSG5 mit einem Marker versieht, mit dem so erhaltenen Hybridplasmid Streptomyceten transformiert, die Transformanten bei einer Temperatur oberhalb von 36°C inkubiert, auf den Markert selektiert und den DNA-Bereich analysiert, über den die Rekombination erfolgte.

## Claims (Claims for the following Contracting State(s): AT, BE, CH, LI, DE, FR, GB, GR, IT, LU, NL, SE)

1. The use of the pSG5 replicon for the preparation of temperature-sensitive hybrid plasmids.

2. A method for finding IS elements and transposons, which comprises providing a plasmid containing the pSG5 replicon with a marker, using the hybrid plasmid obtained in this way to transform Streptomycetes, incubating the transformants at a temperature above 36°C, selecting for the marker, and analyzing the DNA region via which the recombination has taken place.

3. A method for the preparation of Streptomycetes mutants, for the isolation of the mutated genes and for the isolation of the corresponding wild-type gene, which comprises isolating from the starting Streptomycetes strain the complete DNA, converting it into short fragments, integrating these into a hybrid plasmid which has the pSG5 replicon, containing a marker, and transforming the resulting hybrid plasmid population into the starting strain, selecting the transformants by selection for the marker, eliminating the hybrid plasmids by increasing the temperature above the threshold, and selecting the mutants by renewed selection for the marker.

4. The method as claimed in claim 3, wherein the mutated genes are isolated from the selected mutants.

5. The method as claimed in claim 4, wherein the corresponding wild-type genes are isolated using the mutated genes.

6. The method as claimed in claim 4, wherein the hybrid plasmid having the pSG5 replicon contains a marker which can be selected in E. coli, and wherein a cosmid bank is used in the isolation of the mutated genes.

## Claims (Claims for the following Contracting State(s): ES)

1. A method for the preparation of temperature-sensitive hybrid plasmids, which comprises ligating DNA which contains the replicon of pSG5 with foreign DNA.

2. The method as claimed in claim 1, wherein the foreign DNA contains a marker.

3. The method as claimed in claim 1 or 2, wherein the foreign DNA is a linearized plasmid.

4. A method for the preparation of Streptomycetes mutants, for the isolation of the mutated genes and for the isolation of the corresponding wild-type gene, which comprises isolating from the starting Streptomycetes strain the complete DNA, converting it into short fragments, integrating these into a hybrid plasmid which has the pSG5 replicon, containing a marker, and transforming the resulting hybrid plasmid population into the starting strain, selecting the transformants by selection for the marker, eliminating the hybrid plasmids by increasing the temperature above the threshold, and selecting the mutants by renewed selection for the marker.

5. The method as claimed in claim 4, wherein the mutated genes are isolated from the selected mutants.

6. The method as claimed in claim 5, wherein the corresponding wild-type genes are isolated using the mutated genes.

7. The method as claimed in claim 5, wherein the hybrid plasmid having the pSG5 replicon contains a marker which can be selected in E. coli, and wherein a cosmid bank is used in the isolation of the mutated genes.

8. A method for finding IS elements and transposons, which comprises providing pSGS with a marker, using the hybrid plasmid obtained in this way to transform Streptomycetes, incubating the transformants at a temperature above 36°C, selecting for the marker, and analyzing the DNA region via which the recombination has taken place.

## Revendications (Revendications pour l'(les) Etat(s) contractant(s) suivant(s): AT, BE, CH, LI, DE, FR, GB, GR, IT, LU, NL, SE)

1. Utilisation du réplicon de pSG5 pour préparer des plasmides hybrides thermosensibles.

2. Procédé pour détecter des éléments d'insertion et des transposons, caractérisé en ce qu'on fournit un marqueur à un plasmide qui contient le réplicon de pSG5, on transforme des streptomycètes à l'aide du plasmide hybride ainsi obtenu, on incube les produits transformés à une température supérieure à 36°C, on effectue leur sélection sur le critère du marqueur et on analyse le domaine d'ADN dans lequel s'est produite la recombinaison.

3. Procédé pour préparer des mutants de streptomycètes, pour isoler les gènes qui ont muté et pour isoler le gène du type sauvage correspondant, caractérisé en ce qu'on isole l'ADN intégral de la souche de streptomycètes d'origine, on le réduit en fragments courts, on intègre ceux-ci dans un plasmide hybridé avec le réplicon de pSG5, contenant un marqueur, on transforme la population de plasmides hybrides obtenue dans la souche d'origine, on effectue la sélection des produits transformés sur le critère du marqueur, on élimine les plasmides hybrides par augmentation de la température au-dessus de la valeur limite et on sélectionne les mutants au moyen d'une nouvelle sélection sur le critère du marqueur.

4. Procédé selon la revendication 3, caractérisé en ce qu'on isole les gènes qui ont muté dans les mutants sélectionnés.

5. Procédé selon la revendication 4, caractérisé en ce qu'on isole les gènes du type sauvage correspondant à l'aide des gènes qui ont muté.

6. Procédé selon la revendication 4, caractérisé en ce que le plasmide hybridé avec le réplicon de pSG5 contient un marqueur dont la sélection est possible dans E. coli et en ce qu'on utilise une banque de cosmides lors de l'isolation des gènes qui ont muté.

## Revendications (Revendications pour l'(les) Etat(s) contractant(s) suivant(s): ES)

1. Procédé pour préparer des plasmides hybrides thermosensibles, caractérisé en ce qu'on effectue la ligation d'ADN contenant le réplicon de pSG5 avec de l'ADN étranger.

2. Procédé selon la revendication 1, caractérisé en ce que l'ADN étranger contient un marqueur.

3. Procédé selon la revendication 1 ou 2, caractérisé en ce que l'ADN étranger est un plasmide linéarisé.

4. Procédé pour préparer des mutants de streptomycètes, pour isoler les gènes qui ont muté et pour isoler le gène du type sauvage correspondant, caractérisé en ce qu'on isole l'ADN intégral de la souche de streptomycètes d'origine, on le réduit en fragments courts, on intègre ceux-ci dans un plasmide hybridé avec le réplicon de pSG5, contenant un marqueur, on transforme la population de plasmides hybrides obtenue dans la souche d'origine, on effectue la sélection des produits transformés sur le critère du marqueur, on élimine les plasmides hybrides par augmentation de la température au-dessus de la valeur limite et on sélectionne les mutants au moyen d'une nouvelle sélection sur le critère du marqueur.

5. Procédé selon la revendication 4, caractérisé en ce qu'on isole les gènes qui ont muté dans les mutants sélectionnés.

6. Procédé selon la revendication 5, caractérisé en ce qu'on isole les gènes du type sauvage correspondant à l'aide des gènes qui ont muté.

7. Procédé selon la revendication 5, caractérisé en ce que le plasmide hybridé avec le réplicon de pSG5 contient un marqueur dont la sélection est possible dans E. coli et en ce qu'on utilise une banque de cosmides lors de l'isolation des gènes qui ont muté.

8. Procédé pour détecter des éléments d'insertion et des transposons, caractérisé en ce qu'on fournit un marqueur à pSG5, on transforme des streptomycètes à l'aide du plasmide hybride ainsi obtenu, on incube les produits transformés à une température supérieure à 36°C, on effectue leur sélection sur le critère du marqueur et on analyse le domaine d'ADN dans lequel s'est produite la recombinaison.
